(19) **Europäisches Patentamt**

**European Patent Office**

**Office européen des brevets**

(11) **EP 1 147 752 B1**

(12) **EUROPEAN PATENT SPECIFICATION**

(45) Date of publication and mention
of the grant of the patent:
**16.02.2005 Bulletin 2005/07**

(51) Int Cl.[7]: **A61F 7/03**, A61H 33/12,
A61M 16/06, A61M 16/16

(21) Application number: **01109328.3**

(22) Date of filing: **12.04.2001**

(54) **Steam generating unit, mask and eye pillow respectively containing the same, and use thereof**

Dampferzeugungsvorrichtung, Maske bzw. Augenkissen, die es beinhalten und Verwendung desselben

Dispositif de vaporisation, masque et coussin pour les yeux respectivement containant ce dispositif ainsi que son utilisation

(84) Designated Contracting States:
**DE FR GB**

(30) Priority: **18.04.2000 JP 2000117047**
**06.09.2000 JP 2000270685**

(43) Date of publication of application:
**24.10.2001 Bulletin 2001/43**

(73) Proprietor: **Kao Corporation**
**Tokyo 103-8210 (JP)**

(72) Inventors:
• **Yoshihara, Toru, Kao Corporation**
**Tokyo 131-8501 (JP)**
• **Mimura, Koji, Kao Corporation**
**Haga-gun, Tochigi 321-3497 (JP)**
• **Umeda, Tomoshige, Kao Corporation**
**Tokyo 131-8501 (JP)**

• **Fujinami, Susumu, Kao Corporation**
**Tokyo 131-8501 (JP)**
• **Ono, Kenichi**
**Iwatsuki-shi, Saitama 339-0046 (JP)**
• **Mitsuhashi, Tsutomu**
**Iwatsuki-shi, Saitama 339-0046 (JP)**

(74) Representative: **HOFFMANN - EITLE**
**Patent- und Rechtsanwälte**
**Arabellastrasse 4**
**81925 München (DE)**

(56) References cited:
**EP-A- 0 376 490          EP-A- 1 090 614**
**WO-A-99/51174           DE-A- 19 620 534**
**US-A- 3 526 226          US-A- 4 516 564**
**US-A- 5 342 412**

**Description**

SUMMARY OF THE INVENTION

Thereupon, an object of the present invention is to provide a steam generating unit that can be applied safely to any object of application or any site, without the fine particle material leaking.

The inventors conducted assiduous investigations on steam generating units free from leakage of the fine particle material, and, as a result, discovered that, when a sheet exhibiting a certain moisture permeability and gas permeability is used for the configuring material of a bag containing a steam generating composition that uses the heat of oxidation of a metal, steam can be supplied in an adequate volume without powder leakage.

More specifically, the present invention provides a steam generating unit comprising a bag and contained therein a steam generating composition containing metal powder, a salt, and water and releasing steam in conjunction with the oxidation of the metal, wherein the bag comprises a moisture permeable sheet exhibiting a moisture permeability of 1000 $g/m^2$/24h or more but less than 10,000 $g/m^2$/24h as defined by the JIS Z0208 method, and a gas permeability of 30 to 200 seconds/100 $cm^3$ as defined by the JIS P8117 method. use of chemical energy, such as the heat of neutralization of an acid with a base, the heat of hydration of an inorganic salt, or the heat of oxidation of a metal powder such as iron powder, etc. However, when these methods are merely used, the temperature of the steam generated is not controlled, wherefore the direct application thereof to the body involves safety-related problems.

In order to solve these problems, the inventors proposed (in Japanese Patent No. 3049707) a steam generating unit that is used for the skin or mucous membranes, having a steam generator comprising a steam generating composition that uses the heat of oxidation of a metal, where the surface of the generator applied to the skin or mucous membrane comprises a moisture permeable sheet exhibiting a moisture permeability of 4000 $g/m^2$/24h or greater, with the steam release rate from the application surface thereof being 0.01 $mg/cm^2$/min or greater, and the temperature of the steam released controlled to 50°C or below.

However, the steam generating unit of Patent No. 3049707 suffers from a problem in that, when the moisture permeability of the sheet is increased, a small amount of the fine particle material contained in the steam generating composition leaks out.

EP 1 090 614 A, published on April 11, 2001, describes an eye pillow containing a steam generating unit that uses chemical energy. The unit may comprise sheets having a moisture permeability of 600 $g/m^2 \cdot 24$ h or higher, preferably 1,500 to 3,200 $g/m^2 \cdot 24$ h, determined in accordance with ASTM method E-96-80D.

WO 99/51174 discloses a steam generating unit the surface of which is made from a moisture permeable sheet. The moisture permeation of this moisture permeable sheet is more than 4,000 $g/m^2 \cdot 24$ h, determined by ASTM methods.

DE 196 20 534 Al provides a heat generating unit. The material containing said heat generating unit is made of a gas permeable and steam permeable sheet with a water permeability of 50 to 5,000 $g/m^2 \cdot 24$ h according to ASTM E-96-80D.

EP-A-0 376 490 describes a disposable, self-adhesive body warmer containing a heat generating agent.

US 4,516,564 describes a heat generating body suitable for use as a body warmer which can be locally provided and which has at least one air permeable portion.

US 3,526,226 describes a therapeutic inhaling device for the alleviation of pathological alterations of the upper respiratory tract. The device comprises a means to heat a fluid which then evaporates and is delivered to the nose for inhaling.

In a particularly preferable embodiment thereof, the temperature of the steam released from the steam generating unit is controlled to 50°C or lower.

As preferable modes of utilization of this steam generating unit, a mask incorporating that steam generating unit and an eye pillow incorporating that steam generating unit are also provided.

BRIEF DESCRIPTION OF THE DRAWINGS

Fig. 1A is a plane view of a steam generating unit, and Fig. 1B is a cross-sectional view thereof;
Fig. 2A is a perspective view of a mask incorporating a steam generating unit, while Fig. 2B is a cross-sectional view thereof;
Fig. 3A is a plane of a steam generating unit, while Fig. 3B is a cross-sectional view thereof;
Fig. 4A is a cross-sectional view of a mask of the present invention, while Fig. 4B is a front view thereof, and Fig. 4C is a front view of an exhalation valve;
Fig. 5A is a cross-sectional view of a mask of the present invention; while Fig. 5B is a front view thereof; and

Fig. 6A is a cross-sectional view of a mask of the present invention, while Fig. 6B is a front view thereof.

DETAILED DESCRIPTION OF THE INVENTION

The steam generating unit of the present invention can be applied to any object, and in particular can be applied favorably to the skin or mucous membrane. Here, by application of the steam generating unit to the skin or mucous membrane is meant, for example, causing the steam generating unit to contact the skin or mucous membrane by sticking thereto, or deploying the steam generating unit close to the skin or mucous membrane. In the present invention, moreover, by steam released from the steam generating unit is meant that which includes both water that has been vaporized to a vapor state and that consisting of fine water droplets formed by the condensation of such vapor.

The steam generating unit of the present invention contains metal powder, a salt, and water, and uses a steam generating composition that releases steam in conjunction with the oxidation of the metal powder. For the metal powder used in this steam generating composition, iron powder is preferable because it is economical. The iron powder produces an exothermic reaction such as that represented by the following formula, and releases the water in the system as steam.

$$Fe + (3/4)O_2 + (3/2)H_2O \rightarrow Fe(OH)_3 + 96 \text{ kcal}$$

For the iron powder, specifically, cast iron powder, reduced iron powder, electrolytic iron powder, and scrap iron powder, etc., may be used. Of these, such a reduced iron powder is preferable. In order to conduct the oxidation reaction in the heat generating composition efficiently, moreover, it is preferable that the iron powder contain 50 wt.% or more of iron powder having a specific surface area [BET method] of 400 $g/m^2$ or greater. The iron powder mixing proportion in the heat generating composition is preferably 20 to 80 wt.%, and more preferably 30 to 60 wt.%.

For the metal used in the steam generating composition, besides iron powder, powders of metals such as aluminum, zinc, and copper may also be used.

The salt contained in the steam generating composition may be, for example, sodium chloride, potassium chloride, calcium chloride, or magnesium chloride, etc.

The steam generating composition can be made to contain various components such as moisture-retaining agents (vermiculite, calcium silicate, silica gel, porous silica-based substances, alumina, pulp, wood powder, water absorbent polymers, etc.) and reaction promoters (activated carbon, carbon black, graphite, etc.).

The steam generating composition used in the present invention is similar to the compositions used in the heat generating bodies generally referred to as chemical heaters. Chemical heaters, however, are basically configured as warming devices, and are therefore deliberately designed to exhibit suitable air permeability and so that the water necessary to the reaction does not escape from the heat generating unit. For that reason, the bags that contain the heat generating units in chemical heaters, while being configured of air-permeable materials, are not being aggressively configured of moisture-permeable materials. In Japanese Patent Application Laid-Open No. H1-250252/1989, for example, a sheet is used having a moisture permeability of 100 to 400 $g/m^2/24h$ according to the ASTM method (E-96-80D method) (or 200 to 800 $g/m^2/24h$ according to the JIS Z0208 method). In Japanese Patent No. 2136200, an air-permeable sheet is used that exhibits air permeability of 0.1 to 0.8 $\times$ $10^{-4}$ $cm^3/cm^2/sec/Torr$ (or 15000 to 30000 seconds/100 $cm^3$ according to the JIS P8117 method), while in Japanese Patent Application Laid-Open No. H2-149272 an air-permeable sheet is used that exhibits 5000 to 10000 seconds/100 $cm^3$ by the JIS method.

In the present invention, on the other hand, because the steam generating composition is aggressively used as a steam generator, the bag containing the steam generating composition is configured using a moisture permeable sheet that exhibits a certain moisture permeability and gas permeability. Thus the present invention differs greatly from the way in which the same compositions are used in chemical heaters.

The fact that steam is released to the outside during the exothermic reactions of the compositions noted in the foregoing is itself commonly known, and hair curling heat generating units (cf. Japanese Patent Application Laid-Open No. S62-172907) are known which use that steam generating effect and heating effect. However, the temperature of the steam released during the exothermic reactions of those compositions does not control the gas permeability of that composition, and reaches 60°C or above when opened to the atmosphere or placed in a condition approaching that. When the object of application is human hair, there is no problem with steam at 60°C or higher, but with the skin or mucous membranes, there is a danger of damage being caused when steam at 60°C or higher is continuously supplied thereto. Accordingly, in the present invention, when the steam generating unit is applied to the skin or a mucous membrane, the temperature of the steam released from the steam generating unit is controlled to a temperature of 50°C or lower.

In the present invention, when controlling the temperature of the steam released from the steam generating unit to 50°C or lower, the measurement of that temperature is done according to the JIS S4100 (disposable heater

EP 1 147 752 B1

temperature measurement method.

In one mode of temperature control, a temperature regulating material is employed on the bag containing the steam generating composition, so that the temperature of the steam temperature is lowered when the steam released from the steam generating composition passes through the temperature regulating material.

For the material used to configure the temperature regulating material, there can be used (1) woven or nonwoven fabric, (2) paper, synthetic paper, or other paper product, (3) porous film or porous sheet formed of plastic, natural rubber, recycled rubber, or synthetic rubber, (4) foam plastics such as urethane foam, provided with bore holes, or (5) aluminum or other metal foil provided with bore holes, used either singly or in suitable combinations of more than one type.

When using the temperature regulating material to effect temperature control, the temperature regulating material will become a resistance to the passage of the steam, wherefore the type and thickness of the temperature regulating material should be selected so that the prescribed volume of steam is released from the surface of the steam generating unit inclusive of the temperature regulating material.

As another mode of temperature control, a gap may be provided between the bag containing the steam generating composition and the application site of any object of application (such as the skin or a mucous membrane, for example). This gap should be provided so that the distance between the bag containing the steam generating composition and the skin, mucous membrane, or other application site becomes 5 mm or greater. When this distance is less than 5 mm, the temperature control may be inadequate and burns could be caused.

For such a gap, at least the portion of the outer surface of the steam generating unit that is applied to the skin or mucous membrane may be configured with a plastic molding or the like exhibiting good shape retention.

Other temperature control modes include the method of configuring one surface of the bag with a moisture-permeable sheet, and the other surface with a sheet having very fine pores in it capable of passing oxygen, and the method of deploying a poultice layer holding a large volume of water on the outermost side of the surface of the steam generating unit applied to the skin or mucous membrane, and controlling the temperature with the heat capacity of that water. In the former case, the sheet should exhibit a gas permeability of 3000 to 4000 seconds/100 $cm^3$ as defined by the JIS P8117 method. In the later case, the poultice layer should comprise an aqueous gel made with a watersoluble polymer crosslinker.

By suitably altering the quantity of the steam generating composition or the particle size of the particle material configuring the steam generating composition the reaction rate may be adjusted, and thereby the temperature of the steam released from the surface of the steam generating unit may be controlled. The packing density of the steam generating composition will depend on the purpose for which the steam generating unit is used and the site of application, but 0.05 to 5 g per $cm^2$ is preferable, with 0.15 to 2 g per $cm^2$ being more preferred. If the packing density is too low, the steam generating composition will be cooled by the outside air even if it generates heat, and steam will not be adequately released. Conversely, if the packing density is too high, the supply of oxygen to the steam generating composition will become inefficient, and the oxidation reaction will no longer adequately proceed.

A favorable packing ratio for the steam generating composition in the bag is 0.2 to 0.95, where 1 represents the steam generating composition being packed to the full, in cases where the geometric shape of the bag containing the steam generating composition is spherical or ellipsoidal, with 0.3 to 0.8 being preferable. If the packing ratio is too low, steam will hardly be released at all, whereas, conversely, if it is too high, the oxidation reaction will cease to adequately proceed.

In the present invention, from the perspective of steam supply effectiveness when the object of application is skin or a mucous membrane, the amount of steam released from the surface of the steam generating unit applied to the skin or mucous membrane should be 0.01 mg/$cm^2$·min or greater, and preferably 0.5 mg/$cm^2$·min or greater. This steam release amount can be calculated by first removing the steam generating unit from the container that isolates it from the outside air, at room temperature conditions (20°C, 65% RH), and immediately placing it on the upper pan of a balance capable of measuring in units of 1 mg, and measuring the weight after 15 minutes. The calculation is made using formula (1) below, where the weight at the start of the measurements is $Wt_0$ (g), the weight after 15 minutes is $Wt_{15}$ (g), and the area of the portion of the surface of the steam generating unit applied to the skin or mucous membrane is S ($cm^2$).

Steam release amount (mg/$cm^2$·min)

$$= (Wt_0 - Wt_{15}) \cdot 1000/15S \qquad (1)$$

In the present invention, the sheet used for the bag that contains the steam generating composition should exhibit a moisture permeability of 1000 g/$m^2$/24h or greater but 10,000 g/$m^2$/24h or less at a temperature of 40°C and

4

relative humidity of 90%, as provided in JIS Z0208, and a gas permeability of 30 to 200 seconds/100 cm$^3$, as provided in JIS P8117. Preferable ranges are a moisture permeability of 4000 to 8000 g/m$^2$/24h and gas permeability of 30 to 70 seconds/100 cm$^3$. If the moisture permeability is less than 1000 g/m$^2$/24h, a sufficient steam quantity is not obtained. Conversely, if the moisture permeability exceeds 10,000 g/m$^2$/24h, there arises a danger of fine particles leaking from the steam generating composition. If the gas permeability exceeds 200 seconds/100 cm$^3$, moreover, the speed with which the steam released from the steam generating composition passes through the sheet will be slow, whereupon the bag containing the steam generating composition will sometimes swell up and lead to problems in actual use. By keeping the moisture permeability and gas permeability within the ranges noted above, however, the amount of steam release from the steam generating unit surface can be made 0.01 mg/cm$^2$·min or greater, and even 0.5 mg/cm$^2$·min or greater, without causing fine particle leakage or bag swelling.

There is no particular limitation on the material used to configure the sheet having such moisture permeability and gas permeability as noted above. Use may be made, for example, of woven fabric, nonwoven fabric, paper, or synthetic paper, having mixed therein one or more types of fiber selected from among artificial fibers such as nylon, vinylon, polyester, rayon, acetate, acrylic, polyethylene, polypropylene, and polyvinyl chloride, or natural fibers such as pulp, cotton, flax, silk, and animal fur, etc. Alternatively, a gas impermeable film or sheet such as polyethylene, polypropylene, polyamide, polyester, polyvinyl chloride, polyvinylidene chloride, polyurethane, polystyrene, ethylene vinyl acetate copolymer saponification product, ethylene vinyl acetate copolymer, natural rubber, recycled rubber, and synthetic rubber, etc., provided with micropores, may be used. No matter what material the sheet that is used is made of, the moisture permeability and gas permeability can be made to fall within the ranges noted above by suitably adjusting the fine pore size, fine pore density, and weight, etc., of the sheet.

The bag containing the steam generating composition may be configured from a sheet wherein at least a part thereof has the moisture permeability and gas permeability noted above, and it is not absolutely necessary that the entire bag be made from sheet having that moisture permeability and gas permeability. In a bag comprising two surfaces in opposition, for example, one surface thereof may be made from the sheet having the noted moisture permeability and gas permeability, and the other surface made of a material that is not moisture permeable. Thus the steam released from the steam generating composition can be prevented from scattering, and the steam guided efficiently in the prescribed direction. When the entire surface of the bag is made from sheet having the moisture permeability and gas permeability noted, moreover, a non-moisture-permeable sheet may be superimposed on one surface of the bag in order to make the steam release directional.

In the steam generating unit of the present invention, as necessary, a layer containing a cosmetic or drug can be provided, as suitable, according to the properties of and purpose of using such cosmetic or drug. For a cosmetic or drug that is made to be absorbed into the body from the skin or mucous membrane, for example, a poultice or plaster layer containing cosmetic or drug may with good effect be formed on the surface of the steam generating unit applied to the skin or mucous membrane. Highly volatile drugs or cosmetics may be contained in the surface opposite to the surface applied to the skin or mucous membrane, or contained inside the steam generating composition.

Specific examples of cosmetics and drugs include circulation promoters such as acidic mucopolysaccharides, vitamin E, nicotinic acid derivatives, and alkaloid compounds; swelling suppressants such as anthocyanin, vitamin P, and concritic acid; slimming agents such as aminophylline, and inositol; analgesics such as indomethacin, dichlofenac, ketoprofen, dl-camphor, and methyl salicylate; polyols such as glycerin; humectants such as ceramides and collagens; peeling agents consisting of proteases like papain; depilatory agents such as calcium thioglycollate; autonomic nerve regulators such as γ-orizanol; refined oils such as angelica oil, peppermint oil, ilang-ilang oil, coriander oil, sandlewood oil, eucalyptus oil, cedarwood oil, jasmine oil, ginger oil, teatree oil, pine oil, nutmeg oil, patchouli oil, bergamot oil, vetiver oil, palmarosa oil, marjoram oil, turpentine oil, rosewood oil, rosemary oil, lavender oil, and Japanese cedar oil; aromatic components such as geraniol, menthol, citral, citronellol, sionel, α-cedrene, cedrol, terpineol, terpinene, nerol, nerolidol, patchouli alcohol, pinene, phenylethyl alcohol, vetirerol, benzyl acetate, benzyl alcohol, borneol, linalyl acetate, linalool, limonene, and hinokitiol; planet extracts such as aloe extracts, seaweed extracts, chamomile extracts, swertia herb extracts, Japanese angelica root extracts, marronnier extracts, balm mint extracts, hamamelis extracts, eucalyptus extracts, rosemary extracts, and thistle extracts; and silicones such as decamethyl tetrasiloxane, decamethyl cyclopentanesiloxane, octamethyl trisiloxane, and octamethyl cyclotetrasiloxane.

The steam generating unit of the present invention is stored in an airtight bag or airtight container, and taken therefrom for use.

The steam generating unit of the present invention, while releasing a sufficient volume of steam, exhibits no particle powder leakage, and therefore can be applied safely to any object of application and to any site. It can also be used in various applications other than that of supplying steam to skin or mucous membranes.

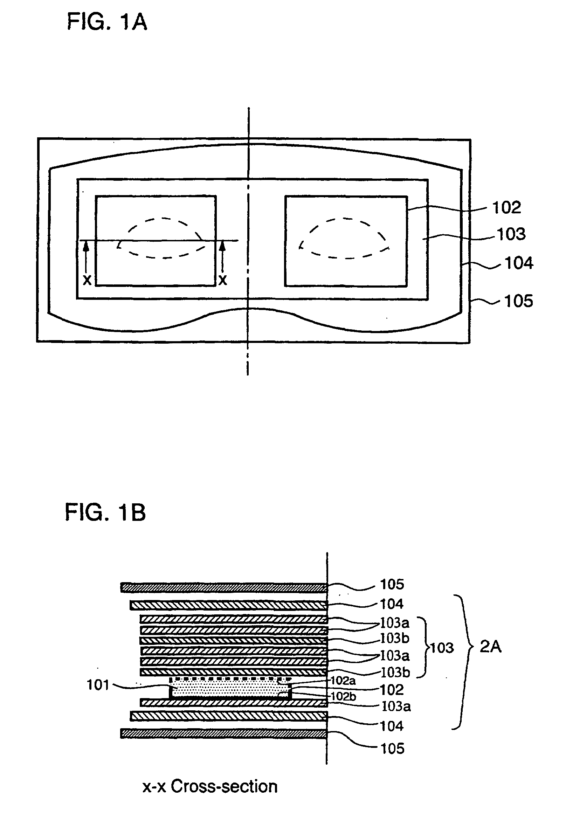

One specific utilization mode for the steam generating unit of the present invention is a mode (cf. Example 1) wherein it is incorporated into an eye pillow, as diagrammed in Fig. 1A and 1B, for example. By incorporating it into an eye pillow, eye dryness that leads to asthenopia and stress resulting from eye overuse can be effectively alleviated, and insomnia and the like prevented.

The steam generating unit of the present invention may also be incorporated into a mask (cf. Examples 2 to 4).

Masks that cover the nose and/or mouth with gauze or the like have been widely used conventionally for preventing colds, and masks are also known which incorporate impregnated substances such as water or drugs (Japanese Patent Application Laid-Open No. H9-99084/1997, Japanese Utility Model Publication No. H5-36422), in none of which is a heating mechanism provided in the mask itself, wherefore the generation of steam from the mask is only promoted by the inhalation action produced by respiration, and it is therewith very difficult to supply steam adequately to the upper respiratory tissue. When a steam generating unit is incorporated into a mask, on the other hand, warmed steam is supplied from the mask, wherefore the upper respiratory tissue can be protected effectively.

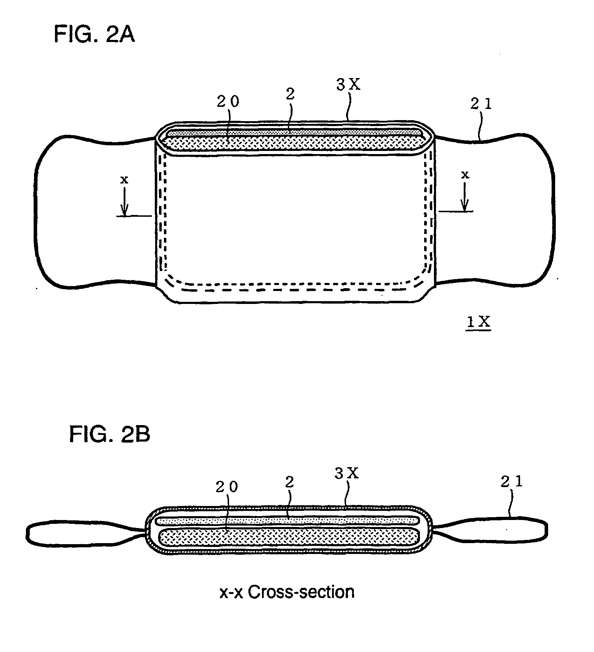

One embodiment of a mask in which a steam generating unit is incorporated is the mask 1X diagrammed in Fig. 2A and 2B, for example, wherein a steam generating unit 2 and a nonwoven cloth 20 acting as a temperature regulating unit are inserted inside a main mask body 3X wherein cotton woven cloth is molded into a bag shape, and rubber ear loops 21 are attached to the main mask body 3X.

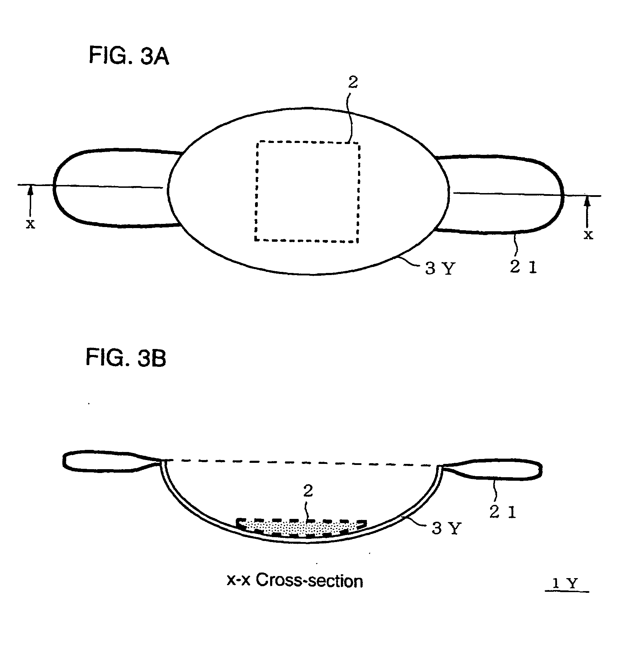

As diagrammed in Fig. 3A and 3B, a molded mask product made of plastic having a semispherical cross-section may be made the main mask body 3Y, and the steam generating unit 2 attached to the inside surface thereof.

When a steam generating unit is provided in a mask, it is preferable that an inhalation valve or exhalation valve be provided in the mask. When that is done, exhaled breath will be exhausted to the outside without stagnating in the main mask body, and the steam released from the steam generating unit can be efficiently inhaled.

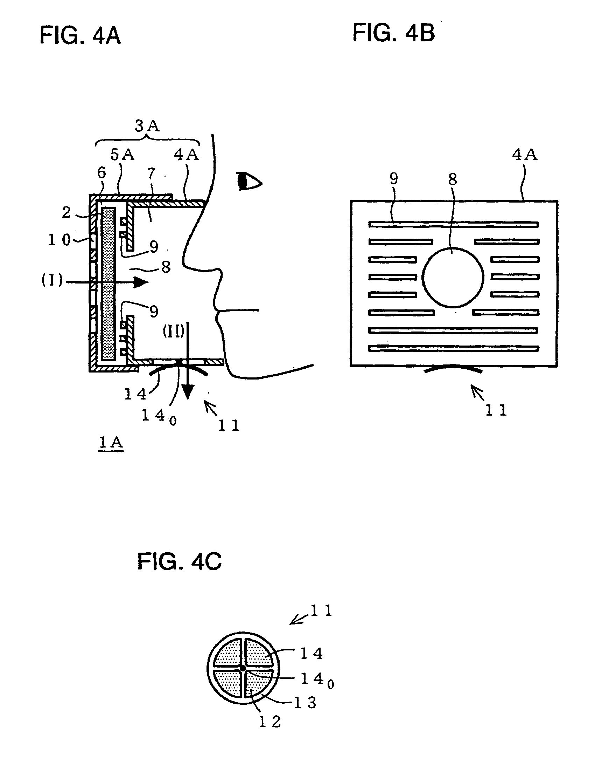

Fig. 4A is a cross-sectional view of such a mask 1A, while Fig. 4B is a front view of the main body part 4A thereof and Fig. 4C is a front view of an exhalation valve 11 used in the mask 1A. This mask-type inhalator 1A comprises a main mask body 3A and a steam generating unit 2 in which the steam generating composition is contained inside a moisture-permeable bag.

The main mask body 3A comprises a cover 5A and a main body part 4A formed as a plastic molded product exhibiting good shape retention. There is no particular limitation on the material used for molding the main body part 4A and cover 5A so long as it exhibits good shape retention, and use may be made of thermoplastic resins such as polypropylenes, polyethylenes, PET, ABS resins, acrylic resins, and vinyl chloride, such thermosetting resins as melamine resins, polyurethane resins, and silicone resins, and such engineering plastics as PBT, polycarbonates, and polyamideimides, etc.

The shape of the main body part 4A is a mask shape that fits tightly against the face, as diagrammed. The main body part 4A and the cover 5A are molded so that, when the cover 5A is attached to the main body part 4A, a space 6 is formed between the main body part 4A and the cover 5A for containing the steam generating unit 2, and so that, when the mask 1a is worn on the face, a gap 7 is formed between the face and the steam generating unit 2 contained in the space 6 described above. Because of this gap 7, the steam released from the steam generating unit 2 can be cooled to a suitable temperature. An inhalation port 8 is opened in the center of the main body part 4A, and ribs 9 are provided around the circumference of the inhalation port 8 in the surface of the main body part 4A that is on the steam generating unit 2 side. The ribs 9 prevent the steam generating unit 2a from tightly pressing against the main body part 4A, and permits the steam released from the steam generating unit 2 to be efficiently inhaled from the inhalation port 8 in the main body part 4A.

In the cover 5A, meanwhile, are provided slits 10 for taking air into the cover 5A. Rims (not shown) are also provided in the surface of the cover 5A, on the steam generating unit 2 side thereof, as in the main body part 4A.

The main body part 4A and cover 5A can be attached and detached. The parts of these that fit together exhibit tight fit, and the only openings in the space 6 that contains the steam generating unit 2 are the inhalation hole 8 and the slits 10 in the cover 5A.

An exhalation valve 11 is deployed in the lower part of the main body part 4A. The exhalation valve 11 operates as a so-called check valve which, when the mask 1A is mounted on the face, closes during inhalation and opens during exhalation. The exhalation valve 11 can be configured as diagrammed in Fig. 4C, for example, such that there are fan-shaped openings 12 in a frame 13, the entire opening 12 is covered by a circular valve disc 14, and the center 140 of the valve disc 14 is secured to the frame 13. There is no particular limitation on the material of the valve disc 14 so long as it is sheet-form material exhibiting pliability. Latex or urethane resins or the like may be used.

When this mask 1A is worn on the face, during inhalation, air flows in the direction of arrow I, whereupon sufficient warm steam can be inhaled through the steam generating unit 2. During exhalation, due to the resistance of the steam generating unit 2A, it is difficult for air to flow toward the steam generating unit 2A, whereupon it flows as indicated by arrow II, and is quickly expelled to the exterior of the main body part 4A. Accordingly, the loss occurring when steam trapped in the space 6 that contains the steam generating unit 2 is expelled due to exhalation can be reduced.

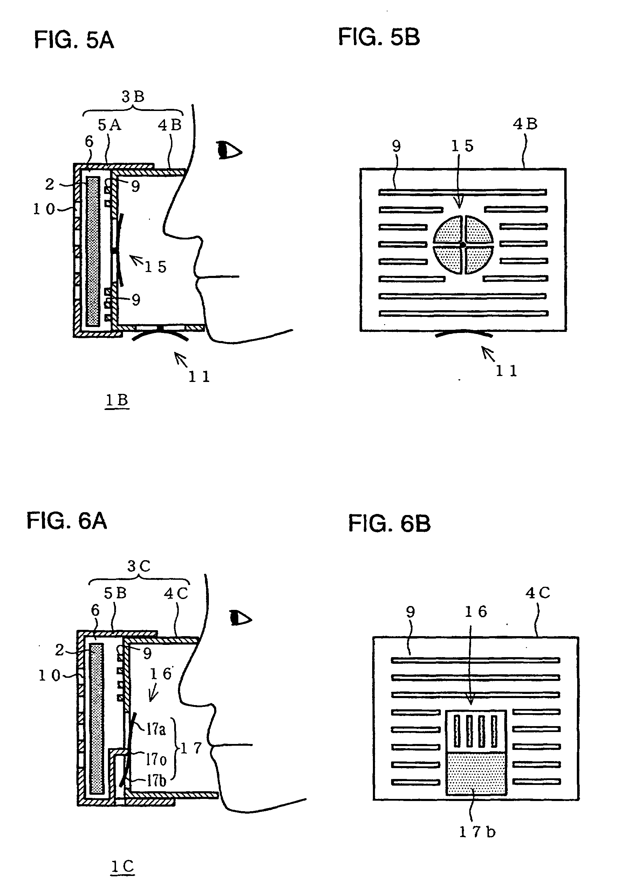

Fig. 5A is a section of a mask 1B in another embodiment of the present invention, and Fig. 5B is a front view of the main body part 4B thereof. This mask 1B has an inhalation valve 15 provided in the main body part 4B instead of the inhalation hole 8 in the mask 1A diagrammed in Fig. 4A. When the mask 1B is worn on the face, the inhalation valve 15 opens during inhalation and closes during exhalation. The inhalation valve 15 used may be equivalent to the exhalation valve 11. By providing this inhalation valve 15, exhaled air is prevented from returning to the space 6 containing the steam generating unit 2, steam can be inspired efficiently through the steam generating unit 2, and exhaled breath can be expelled to the exterior of the mask 1B.

Fig. 6A is a section of a mask 1C of the present invention that is again different, and Fig. 6B is a front view of the main body part 4C thereof. In this mask 1C, a combination inhalation/exhalation valve 16 is provided in the main body part 4C instead of the inhalation hole 8 and exhalation valve 11 of the mask 1A. This combination inhalation/exhalation valve 16 directs the flow of air even better during inhalation and exhalation.

The combination inhalation/exhalation valve 16, more specifically, is configured such that the valve body 17 thereof consists of a pliable sheet, the center $17_0$ of the valve body 17 is secured to the main body part 4C, the upper part $17_a$ of the valve body 17 opens toward the face so that it acts as an inhalation valve, and the lower part $17_b$ thereof is made so that it opens toward the outside (in the direction opposite to the face) so that it acts as an exhalation valve. With this combination inhalation/exhalation valve 16, the functions of both an inhalation valve and an exhalation valve can be elicited from a single valve body 17, which is advantageous in that thereby the space for the valve structure can be reduced and lower cost can be effected.

In this mask 1C, furthermore, the cover 5B is formed in such shape that it divides the lower part $17_b$ of the valve body 17 and the space 6 containing the steam generating unit 2A, without blocking the lower part $117_b$ of the valve body 17, so that exhaled breath will be exhausted to the outside of the mask-type inhalator 1C when the lower part $17_b$ of the valve body 17 is opened to the outside.

The mask of the present invention can take on various other embodiments. In the mask 1B diagrammed in Figs. 5A and 5B, for example, only an inhalation valve 15 may be provided in the main body part 4B, without providing an exhalation valve 11. Even when that is the case, the flow of air during inhalation can be better directed, and warm steam inhaled more efficiently, than in a conventional mask not provided with either an inhalation valve or an exhalation valve.

In the present invention, the material making up the main mask body is not particularly limited so long as it can hold the steam generating unit and is provided with an inhalation valve or an exhalation valve. Accordingly, in the mask 1A diagrammed in Fig. 4A, for example, a bag-shaped member made of a pliable material such as woven fabric, nonwoven fabric, paper, synthetic paper, or film, etc., may be used instead of the molded plastic product that exhibits good shape retention, for the main body part 4A and cover 5A of the main mask body 3A.

In the main mask body, besides the steam generating unit, a drug carrier may be deployed as necessary. There is no particular limitation on the drug type, but one that subdues inflammations in the nose, throat, or other respiratory organs, or that has a so-called aroma therapy effect to relax the nerves or elevate the spirits would be preferable. A drug that is released over a prolonged period by the supply of heat or steam would also be preferable. Examples of such drugs include refined oils or fragrances such as camphor, menthol, peppermint oil, eucalyptus oil, nutmeg oil, turpentine oil, rosemary oil, lavender oil, Japanese cedar oil, cypress thiol, and cedarwood oil. The drug carrier can be formed by causing these drugs to be soaked into a carrier such as paper or nonwoven cloth. The drug carrier can be contained in the space 6 inside the main mask body 3A, in the mask 1A diagrammed in Fig. 4, for example, or incorporated into the main mask body 3A by a commonly known method such as causing it to adhere to the surface of the side of the main body part 4A that is toward the face.

A moisture holding unit that releases steam, other than the steam generating unit, may also be incorporated in the main mask body, as necessary. Paper, nonwoven cloth, woven cloth, or a porous polymer or the like which has been impregnated with water, or a water-absorbing polymer that has been made to absorb water, or the like, can be used for the moisture holding unit. This is desirable because therewith the amount of steam in the air inhaled into the nose and throat can be increased.

Also, a temperature buffer material comprising a woven or nonwoven fabric, paper, synthetic paper, porous film, foam plastic having bore holes, or metal foil having bore holes, or the like, may be deployed in the main mask body, so that the temperature of the steam reaching the face is kept at a safe level, preferably at 50°C or lower, when the mask is mounted on the face.

EXAMPLES

Examples 1 - 6, Comparative Examples 1 - 3

A steam generating composition having the composition noted in Table 1 was prepared. Bags measuring 8 × 8 cm were also prepared, using prototype sheets having the moisture permeability (JIS Z0208 method (40°C, relative humidity 90%) and gas permeability (JIS P8117 method) noted in Table 2, and 10 g of the steam generating composition noted above were placed inside each of these.

Table 1

| Steam generating composition | (unit: wt.%) |
|---|---|
| Water-absorbing polymer<br>    (Aqualick CA, made by Nippon Shokubai) | 5 |
| Activated carbon (made by Takeda Yakuhin) | 4 |
| Vermiculite (made by Shinsei Micron) | 4 |
| 5% saline solution | 36 |
| Iron powder<br>    (product name: RKH, made by Dowa Teppun Kogyo) | 51 |

Table 2

| | Moisture permeability (g/m$^2$/24h) | Gas permeability (seconds/100cm$^3$) |
|---|---|---|
| Example 1 | | |
| Applied Surface(*)<br>Opposite Surface(**) | 7600<br>7600 | 64<br>64 |
| Example 2 | | |
| Applied Surface<br>Opposite Surface | 8000<br>8000 | 52<br>52 |
| Example 3 | | |
| Applied Surface<br>Opposite Surface | 6300<br>6300 | 110<br>110 |
| Example 4 | | |
| Applied Surface<br>Opposite Surface | 4700<br>4700 | 170<br>170 |
| Example 5 | | |
| Applied Surface<br>Opposite Surface | 8000<br>800 | 59<br>5300 |
| Example 6 | | |
| Applied Surface (Opposite Surface made of non-moisture, non-gas permeable sheet (*1)) | 8000 | 59 |
| Comp. Example 1 | | |
| Applied Surface<br>Opposite Surface | 12000<br>12000 | 0.05 (*2)<br>0.05 (*2) |
| Comp. Example 2 | | |
| Applied Surface<br>Opposite Surface | 4300<br>4300 | 255<br>255 |
| Comp. Example 3 | | |
| Applied Surface | 800 | 5300 (*3) |

Note:
(*) Surface applied to skin or mucous membrane
(**) Surface opposite to application surface
(*1) Nitotack, made by Nitto Denko
(*2) Shintex MB, weighed amount 15 g/m$^2$, made by Mitsui Kagaku
(*3) Breslon, made by Nitto Denko

Table 2   (continued)

| | Moisture permeability (g/m$^2$/24h) | Gas permeability (seconds/100cm$^3$) |
|---|---|---|
| Comp. Example 3 | | |
| Opposite Surface | 800 | 5300 (*3) |

(*3) Breslon, made by Nitto Denko

Evaluation

The evaluations noted in (1) to (3) below were made on the steam generating units obtained in Examples 1 to 6 and Comparative Examples 1 to 3. The results are noted in Table 3.

(1) Powder Leak Test

After the steam generating unit was used, it was patted lightly with the palm of the hand, and an observation made with a microscope to determine any presence of powder material leaking out through the sheet. This was evaluated according to the following criteria.

G: No leakage of powder material found
NG: Leakage of powder material found

(2) The bag was observed with the naked eye to determine whether or not there was any swelling while the steam generating unit was generating heat, and evaluations were made according to the following criteria.

A: No swelling found
B: Slight swelling found; no problem in actual use
C: Pronounced swelling; unsuitable for actual use

(3) Steam Release Amount

The steam release amounts determined by formula (1) given earlier were determined.

Table 3

| | Powder material Leakage | Bag swelling | Steam release amount (mg/cm$^2$/min) |
|---|---|---|---|
| Ex. 1 | G | A | 0.90 |
| Ex. 2 | G | A | 1.15 |
| Ex. 3 | G | A | 1.19 |
| Ex. 4 | G | B | 1.25 |
| Ex. 5 | G | A | 1.21 |
| Ex. 6 | G | B | 1.26 |
| Comp. Ex. 1 | NG | A | 1.32 |
| Comp. Ex. 2 | G | C | 0.89 |
| Comp. Ex. 3 | G | A | 0.06 |

From the results given in Table 3, it will be seen that the steam generating bodies in Examples 1 to 6 can be applied to any site because they exhibit no powder leakage and no swelling that would be problematic in actual use, and adequately release steam. It will also be seen that, in contrast to this, powder leakage occurs in Comparative Example 1, where the moisture permeability exceeds 10000 g/m$^2$/24h, that the bag swells with the moisture-permeable sheet of Comparative Example 2, where the gas permeability exceeds 200 seconds/100 cm$^3$, and that, while neither powder leakage nor bag swelling occurs, the steam release amount is low in Comparative Example 3, where the moisture permeability is lower than 1000 g/m$^2$/24h and the gas permeability exceeds 200 seconds/100 cm$^3$.

Example 7

Two 3 cm x 3 cm square bags were fabricated, using moisture-permeable sheet 102a exhibiting a moisture permeability of 7800 g/m$^2$/24h and a gas permeability of 36 seconds/100 cm$^3$ for one surface, and non-moisture-permeable sheet (Nitotack, made by Nitto Denko) 102b for the other surface, and these were each filled with the same steam generating composition 101 as in Example 1.

As diagrammed in Fig. 1A and 1B, the two bags 102 filled with the steam generating composition 101 were bonded securely to the nonwoven cloth 103a (Airaid, weighed amount 24 g/m$^2$, made by Chisso), with the surface of the non-moisture-permeable sheet 102b as the supporting base, aligning side by side an interval for the eyes, a temperature regulating material 103 (comprising one layer of paper 103b (Kimtowel, made by Kureshia), two layers of nonwoven cloth 103a (Airaid, weight amount 24 g/m$^2$, made by Chisso), 1 layer of paper 103b, and 2 layers of nonwoven cloth 103a) were provided on the surface of the moisture-permeable sheet 102a, the entirety thereof was placed in an outer bag 104 ( Syntex MB, made by Mitsui Kagaku) comprising a moisture-permeable nonwoven cloth fabricated in an eye mask shape, and the entirety of the outside thereof was sealed in an airtight bag 105 (Hiryu, made by Asahi Kasei Polyflex).

60 seconds after this steam generating unit 2A was removed from the airtight bag 105, steam at a temperature of 40 to 42°C was released for 10 minutes. During that time, there was no swelling of the bag 102. After the steam release was finished, no powder material was found to be adhering to the surface of the outer bag 104 comprising the moisture-permeable nonwoven cloth.

Example 8

The steam generating composition having the composition given in Table 4 was prepared.

Table 4

| Steam generating composition | (unit: wt.%) |
|---|---|
| Water-absorbing polymer (Aqualick CA, made by Nippon Shokubai) | 5 |
| Activated carbon (made by Takeda Yakuhin) | 4 |
| Vermiculite (made by Shinsei Micron) | 4 |
| 5% saline solution | 36 |
| Lavender oil | 0.4 |
| Nonionic surfactant | 0.4 |
| Iron powder (product name: RKH. made by Dowa Teppun Kogyo) | 50.2 |

A bag measuring 8 x 8 cm was also prepared, using sheet having the moisture permeability (JIS Z0208 method (40°C, relative humidity 90%) of 8200 g/m2/24h and gas permeability (JIS P8117 method) of 25 seconds/100 cm$^3$, and 25 g of the steam generating composition noted above were placed therein to make the steam generating unit 2, which was then sealed in an airtight bag.

Meanwhile, rubber ear loops 21 were attached to the opposite ends of a main mask body 3Y comprising a molded product made of polypropylene having a semispherical cross-section, as diagrammed in Figs. 3A and 3B.

The steam generating unit 2 (bag filled with the steam generating composition) described above was removed from the airtight bag and immediately pressed against and secured to the inner surface of the main mask body 3Y, yielding the mask 1Y incorporating the steam generating unit. When this mask 1Y was donned, there was a distance of approximately 3 cm between the face and the surface of the steam generating unit 2.

With this mask 1Y, 60 seconds after the steam generating unit 2 was removed from the airtight bag, steam at a temperature of 40 to 45°C was released for 10 minutes. During that time there was no swelling of the steam generating unit. After the steam release was finished, no powder material was found adhering to the surface of the steam generating unit.

Example 9

The mask 1A of the embodiment diagrammed in Fig. 4A was fabricated as follows.

First, a water-absorbent polymer (product name: Aqualick, made by Nihon Shokubai), vermiculite (made by Shinsei Micron), and activated carbon (product name: Carbolifin, made by Takeda Yakuhin) were mixed in the mixing ratio indicated in Table 5. Into this mixed powder was mixed a mixture solution wherein lavender oil and 5 wt.% of saline solution was dispersed with a nonionic activator (product name: Softanol 90, made by Nihon Shokubai). Then iron powder (product name: RKH, made by Dowa Teppun Kogyo) was mixed in to yield a steam generating composition.

This steam generating composition was placed inside a moisture-permeable bag (material: melt-blown polypropylene nonwoven cloth, size: 90 x 74 mm), thus fabricating the steam generating unit 2.

For the main body part 4A of the main mask body 3A, a plastic molded product (size: 84 x 75 mm) having an inhalation hole 8 of diameter 27 mm in one surface thereof, made of polypropylene, and provided with a valve body 14 made of natural rubber in the bottom surface thereof, was fabricated. For the cover 5A, a molded product of the same polypropylene was fabricated so as to fit together with the main body part 4A.

The steam generating unit 2 described above was contained inside the space 6 between the cover 5A and main body part 4A of this main mask body 3A to form the mask 1A

Table 5

|  | (Unit: wt.%) |
|---|---|
| Water-absorbent polymer | 5 |
| Activated carbon | 4 |
| Vermiculite | 4 |
| 5 wt.% saline solution | 36 |
| Lavender oil | 0.4 |
| Nonionic surfactant | 0.4 |
| Iron powder | 50.2 |

Example 10

A mask 1B of the embodiment diagrammed in Fig. 5A was fabricated.

In this case, the steam generating unit 2 was fabricated in the same way as in Example 9. The main mask body 3B was fabricated with an inhalation valve 15 having a valve body made of natural rubber deployed at the position of the inhalation hole 8 in the main body part 4A of Example 9. The steam generating unit 2 was contained inside the space 6 between the cover 5A and main body part 4A of the main mask body 3B to form the mask 1B.

Example 11

A mask 1C of the embodiment diagrammed in Fig. 6 was fabricated.

In this case, for the steam generating unit 2 fabricated, in preparing the steam generating composition of Example 9, eucalyptus oil was used instead of lavender oil.

For the main mask body 3C fabricated, instead of the inhalation hole 8 and exhalation valve 11 in the main body part 4A of Example 9, an inhalation-exhalation valve 16 having a natural rubber valve body 17 secured to the main body part 4c in the center part $17_0$ thereof was deployed. The steam generating unit 2 described earlier was contained inside the space 6 between the cover 5B and the main body part 4C of this mask-form face contacting part 3C to make the mask 1C.

Comparative Example 4

A commercially available gauze mask was prepared.

Evaluation

Functional tests using Examples 9 to 11 and Comparative Example 4 were conducted with 10 human monitors. Evaluations were obtained from each monitor according to the following criteria. The results are given in Table 6.

Evaluation Criteria

A: Inhalation of steam could be adequately sensed
B: Inhalation of steam was sensed, but inhalation volume was felt to be inadequate
C: Inhalation of steam could hardly be sensed at all

Table 6

| | Monitor | | | | | | | | | |
|---|---|---|---|---|---|---|---|---|---|---|
| | A | B | C | D | E | F | G | H | I | J |
| Ex. 9 | A | A | A | A | A | A | A | A | A | A |
| Ex. 10 | A | A | A | A | A | A | A | A | A | A |
| Ex. 11 | A | A | A | A | A | A | A | A | A | A |
| Comp. Ex. 4 | C | C | B | C | C | C | C | C | C | C |

From the results in Table 6, it is seen that the inhalation of steam can be sensed when the example masks of the present invention having an inhalation valve or exhalation valve are used, but that, with the commercially available gauze mask of Comparative Example 4, the inhalation of steam can hardly be sensed at all because only the moisture contained in the exhaled breath is the steam supply source.

## Claims

1. A steam generating unit, comprising a bag (102) and contained therein a steam generating composition (101) containing metal powder, a salt, and water, and releasing steam in conjunction with oxidation of said metal powder, wherein said bag (102) comprises a moisture-permeable sheet (102a) exhibiting a moisture permeability of 1000 $g/m^2/24h$ or higher but no greater than 10000 $g/m^2/24h$, as defined by JIS Z0208, and a gas permeability of 30 to 200 seconds/100 $cm^3$, as defined by JIS P81117.

2. A mask (1X, 1Y, 1A, 1B, 1C), comprising the steam generating unit according to claims 1 incorporated therein.

3. The mask (1A) according to claim 2, wherein an inhalation valve (15) or exhalation valve (11) is provided.

4. The mask (1B) according to claim 3, wherein said inhalation valve (15) and said exhalation valve (11) are provided separately.

5. The mask (1C) according to claim 3, wherein a combination inhalation/exhalation valve (16) is provided.

6. An eye pillow, comprising the steam generating unit according to claim 1 incorporated therein.

7. The eye pillow according to claim 6, wherein exterior shape is shaped in form of an eye mask.

## Patentansprüche

1. Dampfgenerierende Einheit, umfassend eine Tasche (102) und, hierin enthalten, eine dampfgenerierende Zusammensetzung (101), enthaltend Metallpulver, ein Salz und Wasser, die Dampf im Zusammenhang mit der Oxidation des Metallpulvers freisetzt, wobei die Tasche (102) eine feuchtigkeitsdurchlässige Lage (102a) umfasst, die eine Feuchtigkeitsdurchlässigkeit von 1.000 $g/m^2/24$ h oder mehr, aber nicht mehr als 10.000 $g/m^2/24$ h, definiert gemäss JIS Z0208, und eine Gasdurchlässigkeit von 30 bis 200 Sekunden/100 $cm^3$, definiert gemäss JIS P8117, aufweist.

2. Maske (1X, 1Y, 1A, 1B, 1C), umfassend hierin eingearbeitet die dampfgenerierende Einheit gemäss Anspruch 1.

3. Maske (1A) nach Anspruch 2, die mit einem Einatmungsventil (15) oder einem Ausatmungsventil (11) ausgestattet ist.

4. Maske (1B) nach Anspruch 3, wobei das Einatmungsventil (15) und das Ausatmungsventil (11) getrennt bereitgestellt werden.

5. Maske (1C) nach Anspruch 3, wobei eine Kombination Einatmungs-/Ausatmungsventil (16) bereitgestellt wird.

**EP 1 147 752 B1**

**6.** Augenkissen, umfassend hierin eingearbeitet die dampfgenerierende Einheit nach Anspruch 1.

**7.** Augenkissen nach Anspruch 6, wobei die äussere Form in Form einer Augenmaske geformt ist.

**Revendications**

**1.** Unité de génération de vapeur, comprenant un sac (102) et, contenue à l'intérieur de celui-ci, une composition (101) de génération de vapeur comprenant du métal en poudre, un sel et de l'eau, et libérant de la vapeur en association avec l'oxydation dudit métal en poudre, dans laquelle ledit sac (102) comprend une feuille (102a) perméable à l'humidité présentant une perméabilité à l'humidité égale ou supérieure à 1000 g/m$^2$/24h, mais ne dépassant pas 10000 g/m$^2$/24h, comme il est défini par JIS Z0208, et une perméabilité aux gaz de 30 à 200 secondes/100 cm$^3$, comme il est défini par JIS P8117.

**2.** Masque (1X, 1Y, 1A, 1B, 1C), incorporant l'unité de génération de vapeur selon la revendication 1.

**3.** Masque (1A) selon la revendication 2, dans lequel est prévue une soupape d'inspiration (15) ou une soupape d'expiration (11).

**4.** Masque (1B) selon la revendication 3, dans lequel ladite soupape d'inspiration (15) et ladite soupape d'expiration (11) sont fournies séparément.

**5.** Masque (1C) selon la revendication 3, dans lequel est fournie une combinaison de soupape d'inspiration et d'expiration (16).

**6.** Coussin de protection des yeux, incorporant l'unité de génération de vapeur selon la revendication 1.

**7.** Coussin de protection des yeux selon la revendication 6, dans lequel la forme extérieure a la forme d'un masque oculaire.

13

## FIG. 1A

## FIG. 1B

x-x Cross-section

## FIG. 2A

## FIG. 2B

x-x Cross-section

## FIG. 3A

## FIG. 3B

x-x Cross-section

1 Y

## FIG. 4A

## FIG. 4B

## FIG. 4C

FIG. 5A

FIG. 5B

FIG. 6A

FIG. 6B